# EUROPEAN PATENT SPECIFICATION

(11) **EP 1 104 307 B1**
(45) Date of publication and mention of the grant of the patent: **07.06.2006**
(21) Application number: 99935073.9
(22) Date of filing: 05.08.1999
(51) Int. Cl.: A61K 39/395, A61K 38/17, A61K 31/70, A61K 31/739, A61P 13/12

(54) **PHARMACEUTICAL COMPOSITION HAVING INHIBITORY EFFECT ON OVERPRODUCTION AND ACCUMULATION OF EXTRACELLULAR MATRIX**
PHARMAZEUTISCHE ZUSAMMENSETZUNG DIE EIN INHIBIERENDES EFFEKT AUF DIE ÜBERPRODUKTION UND AKKUMULATION DER EXTRAZELLULÄREN MATRIX HAT
COMPOSITION PHARMACEUTIQUE A ACTION INHIBITRICE DE LA SURPRODUCTION DE MATRICE EXTRACELLULAIRE ET DE SON ACCUMULATION

(30) Priority: 06.08.1998 JP 23349998
(43) Date of publication of application: 06.06.2001
(73) Proprietor: TEIJIN LIMITED, Osaka-shi Osaka 541-0054 (JP)
(72) Inventor: SASAKI, Satoshi Teijin Limited, Tokyo Res. Ctr., Hino-shi, Tokyo 191-0065 (JP); SUMI, Yoshihiko Teijin Limited, Tokyo Res. Ctr., Hino-shi, Tokyo 191-0065 (JP); HUGHES, Reginald Colin National Inst. Med. Res., London, Greater London NW7 1AA (GB)
(74) Representative: Hallybone, Huw George
(86) International application number: PCT/JP1999/004238
(87) International publication number: WO 2000/007624

(56) References cited:
- OCHIENG J ET AL: "Regulation of cellular adhesion to extracellular matrix proteins by galectin-3." BIOCHEMICAL AND BIOPHYSICAL RESEARCH COMMUNICATIONS, (1998 MAY 29) 246 (3) 788-91. , XP002131316
- HENRICK K ET AL: "Evidence for subsites in the galectins involved in sugar binding at the nonreducing end of the central galactose of oligosaccharide ligands: sequence analysis, homology modeling and mutagenesis studies of hamster galectin-3." GLYCOBIOLOGY, (1998 JAN) 8 (1) 45-57. , XP000876954
- PROBSTMEIER R ET AL: "Galectin-3, a beta-galactoside-binding animal lectin, binds to neural recognition molecules." JOURNAL OF NEUROCHEMISTRY, (1995 JUN) 64 (6) 2465-72. , XP000876914
- LIU F T ET AL: "Modulation of functional properties of galectin -3 by monoclonal antibodies binding to the non-lectin domains." BIOCHEMISTRY, (1996 MAY 14) 35 (19) 6073-9. , XP002131317 cited in the application
- GRANSTEIN R.D. ET AL J. INVESTIGATIVE DERMATOLOGY vol. 95, no. 6 SUPPL, December 1990, pages 75S - 80S, XP001183478
- KANG H.-S. CELLULAR IMMUNOLOGY vol. 170, 1996, pages 212 - 221, XP001183477
- FULLERTON M.J.; FUNDER J.W. CARDIOVASCULAR RESEARCH vol. 28, 1994, pages 1863 - 1867, XP009036223
- ZIYADEH F.N. ET AL J. CLINICAL INVESTIGATION vol. 93, February 1994, pages 536 - 542, XP009036217

## Description

### FIELD OF INVENTION

The present invention relates to a preventive or therapeutic pharmaceutical composition having an inhibitory effect on the overproduction and the accumulation of extracellular matrix, said composition comprising as an active ingredient a compound that inhibits the biological activity of galectin-3.

### BACKGROUND ART

Galectin-3 is a protein that has a molecular weight of about 30 Kd belonging to the family of β-galactoside-binding protein and is a lectin that widely occurs on the cell surface, the cytoplasm and the nucleus of the tissue (see, for example, Barondes, S. H: et al., J. Biol. Chem. (1994) 296: 20807-20810, Hughes, R. C. Glycobiology (1994) 4: 5-12, Wang, L. et al., Biochem. Biophys. Res. Commun. (1995) 217: 292-303, and the like). It is known that galectin-3 binds to a suitable sugar chain portion of glycoprotein present on the cell surface or in the extracellular matrix (ECM) and thereby activates inflammatory cells such as neutrophils, basophils, or macrophages to promote the production of cytokines from these cells (see, for example, Sato, S. et al., J. Biol. Chem. (1994a) 269: 4424-4430, Liu, F. T. Immunol. Today (1993) 14: 486-490), or to suppress the apoptotic death of T cells by its overexpression (see Yang, R-Y, H. et al., Proc. Natl. Acad. Sci. U.S.A. (1996) 93: 6737-6742), and it is believed to be an important protein responsible for inflammatory and immunological reactions. It has also been suggested that cell surface-exposed galectin-3 is involved in the formation of cell aggregates (see Probstmeier, R. et al., J. Neurochemistry (1995) 64(6):2465-2472).

Furthermore, galectin-3 is also known to play an important role in the formation and repair of the tissue since it is highly expressed during the damage repair period in the rat lung-injured model induced by X-ray irradiation (see Kasper, M. et al., J. Pathol. (1996) 179: 309-316) and it is possibly playing an important role in the formation of kidney tissue in humans during the embryonic stage (see Winyard, P. J. D. et al., J. Am. Soc. Nephrol. (1997) 8: 1647-1657).

The overproduction and the accumulation of extracellular matrix (ECM) such as collagen is believed to be an important factor for the pathogenesis of the fibrosis of tissues such as liver, kidney, lung, heart, pancreas, artery, gastrointestinal tract, thyroid, salivary gland, and skin (see Okada, H. et al., Kidney Int. (1996) 49: Supple. 54: S-37-S-38, Coker, R, K. et al., Eur. Respir. J. (1998) 11(6) : 1218-1221 and the like). ECM is also involved in the maintenance of homeostasis of cellular functions together with the support of parenchymal cells at the physiological conditions. When minor injuries are inflicted to tissues, the repair of the injured tissues is completed by the treatment of the injured tissues by phagocytic cells in the process of inflammatory and repair reactions, the subsequent regeneration of parenchymal cells, and the reconstruction of the supportive substrate, ECM. However, when the injuries are severe or persist for a long time, the overproduction and the accumulation of ECM will cause severe damages in the functions of each tissue. In the liver, for example, lymphocytes and macrophages infiltrate to the periphery of the injured liver cells, and these cellular infiltrates and Kupfer cells or vascular endothelial cells and the like produce cytokines such as PDGF, TGF-β, etc., which then activate Ito cells, a kind of ECM-producing cells. The activated Ito cells proliferate and produce ECM in an excess amount in the Disse's space thereby to cause hepatic fibrosis or hepatic cirrhosis that are said to be a terminal status of the hepatic diseases. In the kidney glomeruli, for example, cytokines such as PDGF and TGF-β are produced from the cells that have infiltrated into the periphery of the injured kidney cells or endothelial cells in the glomeruli etc. to activate the mesangium cells that are a kind of ECM-producing cells. The activated mesangium cells proliferate while themselves also producing cytokines such as PDGF and TGF-β together with an excessive amount of ECM, creating factors that cause various glomerular diseases, for example, chronic glomerular nephritis, including IgA nephropathy, diabetic nephropathy, glomerular sclerosis and the like. In the interstitial tissue of the kidney also, due to the activation of myofibroblasts, a kind of ECM-producing cells, and the epithelial cells of urinary tubules, these cells excessively produce ECM in the interstitial region of the urinary tubules and form fibrosis of the tubulo-interstitium thereby significantly reducing the renal function. Thus, the ECMs that were overproduced and accumulated in each tissue physically constrain the cellular functions of each cell and substitute for the functional unit of each tissue to cause severe functional disorders of each organ.

For the above-mentioned diseases, adrenal cortical steroids, immunosuppressive agents, anti-platelet agents, anti-coagulants, anti-fibrinolytic agents, ACE inhibitors, and the like are currently used, but no drugs exhibit satisfactory efficacy on the overproduction and the accumulation of ECM, and there is a strong need for agents that have a novel mechanism of action.

Although galectin-3 has been highly expressed at the injured site of the tissue in the rat lung-injured models induced by X-ray irradiation, a model of pulmonary fibrosis, it has not been elucidated whether it can regulate the overproduction and the accumulation of ECM such as collagen and the survival of the mesangium cells that are a kind of ECM-producing cells. Accordingly, it is not known whether the inhibition of the action of galectin-3 can inhibit the overproduction and the accumulation of ECM and thereby it has a therapeutic and/or preventive usefulness for glomerular nephritis, diabetic nephropathy or tissue fibrosis.

### DISCLOSURE OF INVENTION

It is on object of the present invention to provide the use of a compound that binds galectin-3 in the manufacture of a medicament for treating glomerular nephritis, diabetic nephropathy or tissue fibrosis as defined in the claims.

Considering the state of art of the conventional technology, the present inventors have carried out intensive study and have found that galectin-3 is a molecule that can regulate the overproduction and the accumulation of ECM such as collagen and a molecule that can regulate the survival of the mesangium cells which is a kind of ECM-producing cells, and also have found that substances that inhibit the biological activity of galectin-3 can regulate the overproduction and the accumulation of ECM such as collagen.

This indicates that compounds that inhibit the biological activity of galectin-3 can be a therapeutic or preventive agent of glomerular nephritis, diabetic nephropathy or tissue fibrosis of which cause is the overproduction and the accumulation of extracellular matrix.

### BRIEF EXPLANATION OF THE DRAWINGS

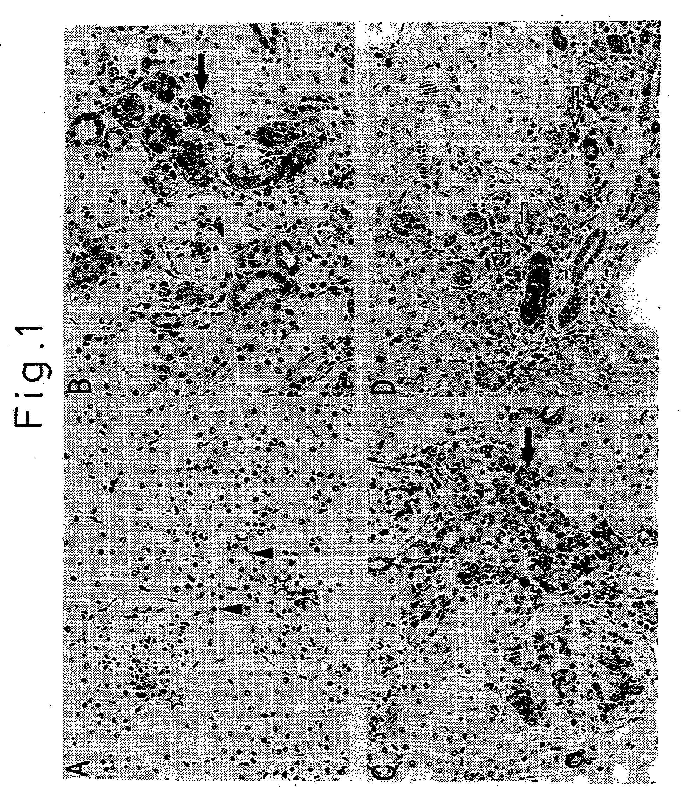

Figure 1 shows a variation in the expression of galectin-3 in the anti-Thy-1.1 antibody-induced rat nephritis model.
   A: the kidney of the rats who received no anti-Thy-1.1 antibody, B: day 8 after the administration of anti-Thy-1.1 antibody, C and D: day 14 after the administration of anti-Thy-1.1 antibody.
   The asterisk in the figure shows a representative macula densa region, m a representative mesangium region, c a representative crescent body-forming region, the closed arrow tail a representative distal urinary tubule, the closed arrow a representative proximal urinary interstitial tubule, and the open arrow a representative infiltrated macrophage or fibroblast.
Figure 2 shows a variation in the expression of galectin-3 in the UUO rat model.
   A: contralateral kidney, B: obstructed kidney
   The asterisk in the figure shows a representative macula densa region, the closed arrow tail a representative distal urinary tubule, and the open arrow a representative infiltrated macrophage or fibroblast.
Figure 3 shows the activity of galectin-3 to inhibit the cellular death of the mesangium cells.
Figure 4 shows the activity of galectin-3 to promote the production of collagen type IV by the rat mesangium cells.
Figure 5 shows the suppression by galectin-3 binding-inhibiting glycoprotein of the activity of galectin-3 to promote the production of collagen type IV production by the rat mesangium cells.
Figure 6 shows the suppression by galectin-3 binding-inhibiting sugar of the activity of galectin-3 to promote the production of collagen type IV production by the rat mesangium cells.

### DETAILED DESCRIPTION

Compounds that bind to galectin-3 for use in the present invention include, for example, the following:
(1) Anti-galectin 3 antibody: mouse anti-galectin 3 monoclonal antibody (for example, an antibody described in Lui, F. T., Et al., J. Biol. Chem. (1996) 35: 6073-6079);
(2) Inhibitors of galectin-3 binding: sugars to which galectin-3 can bind such as Galβ1-4Glc, Galβ1-4GlcNAc, Fucα1-2Galβ1-4Glc, Galα1-3Galβ1-4GlcNAc Galβ1-3GlcNAcβ1-3Gaβ1-4Glc, Galβ1-4GlcNAcβ1-3Galβ1-4Glc, Fucαl-2Galβ1-3GlcNAcβ1-3Galβ1-4Glc, Galβ1-3(Fucα1-4) GlcNAcβ1-3Ga1β1-4Glc, Galβ1-4(Fucα1-3)GlcNAβ1-3Gaβ1-4Glc, Galβ1-3GlcNACβ1-3Ga1β1-4(Fucα1-3)Glc, Fucα1-2(GlcNAcal-3)Galβ1-3GlcNAcβ1-3Galβ1-4Glc, NeuNAcα2-3Galβ1-3GlcNAcβ1-3Galβ1-4Glc, NeuNAcα2-6Galβ1-4GlcNACβ1-3Galβ1-4Glc, Galβ1-3(NeuNAcα2-6)GlcNAcβ1-3Galβ1-4Glc, Galβ1-3GlcNAcβ1-3Galβ1-4GlcNAcβ1-3Galβ1-4Glc, Galβ1-4GlcNAcβ1-3Galβ1-4GlcNAcβ1-3Galβ1-4Glc, Galβ1-3GlcNAcβ1-3Galβ1-4(Fucα1-3)GlcNAcβ1-3Galβ1-4Glc, Galβ1-4GlcNAcβ1-6(Galβ1-3GlcNAcβ1-3)Galβ1-4Glc, Galβ1-4GlcNAcβ1-6(Galβ1-4GlcNAcβ1-3)Galβ1-4Glc, Galβ1-4GlcNAcβ1-6(Galβ1-4GlcNAcβ1-2)Manα1-6(Galβ1-4GlcNAcβ1-2Manα1-3)Manβ1-4GlcNAc, Galβ1-4GlcNAcβ1-2Manα1-6(Galβ1-4GlcNAcβ1-4(Galβ1-4GlcNAcβ1-2)Manα1-3)Manβ1-4GlcNAc, GlcNAcβ1-3Galβ1-4GlcNAcβ1-3Galβ1-4Glc, Galα1-3Galβ1-4GlcNAcβ1-3Galβ1-4Glc, GalNAcα1-3(Fucα1-2)Galβ1-3GlcNAcβ1-3Galβ1-4Glc, Galα1-3(Fucα1-2)Galβ1-3GlcNAcβ1-3Galβ1-4Glc, Galβ1-4GlcNAcβ1-6(Galβ1-4GlcNAcβ1-3)Galβ1-4GlcNACβ1-3Galβ1-4Glc, Galα1-3Galβ1-4GlcNAcβ1-6(Galα1-3Galβ1-4GlcNAcβ1-3)Galβ1-4GlcNAcβ1-3Galβ1-4Glc, Galβ1-4GlcNAcβ1-6(Galβ1-4GlcNAcβ1-3)Galβ1-4GlcNAcβ1-6(Galβ1-4GlcNAcβ1-3)Galβ1-4GlcNAcβ1-3Galβ1-4Glc, Galα1-3Galβ1-4GlcNAcβ1-6(Galα1-3Galβ1-4GlcNAcβ1-3)Galβ1-4GlcNAcβ1-6(Galα1-3Galβ1-4GlcNAcβ1-3)Galβ1-4GlcNacβ1-3Galβ1-4Glc, Galβ1-4GlcNAcβ1-2Manα1-6(Galβ1-4GlcNAcβ1-2Manα1-3)Manβ1-4GlcNAc, Galβ1-4GlcNAβ1-2Manα1-6(Galβ1-4GlcNAcβ1-4(Galβ1-4GlcNAcβ1-2)Manα1-3)Manβ1-4GlcNAc, Galβ1-4GlcNAcβ1-6(Galβ1-4GlcNAcβ1-2)Manα1-6(Galβ1-4GlcNAcβ1-4(Galβ1-4GlcNAcβ1-2)Manα1-3)Manβ1-4GlcNAc, and blood type B-like sugar chains, or glycolipids comprising the above sugars, or glycoproteins having on the cell surface sugar chains to which galectin-3 can bind, or fragments thereof such as fetuin, asialofetuin, transferrin, asialotransferrin, α1-acid glycoprotein, asialo α1-acid glycoprotein, laminin, fibronectin, CD11b, Lamp-1, Lamp-2, Mac-3, CD98, neutrophil 115 kD protein, neutrophil 180 kD protein (NCA-160/CD66), high-affinity IgE receptor, Fc ε Rl, and the like (see, for example, Feizi, T. et al., Biochemistry (1994) 33: 6342-6349, Sato, S., et al., J. Biol. Chem. (1992) 267: 6983-6990).

Compounds that bind galectin-3 for use in the present invention can be formulated to make pharmaceutical compositions having an inhibitory effect on the overproduction and the accumulation of ECM by blending said compounds as active ingredients and pharmaceutically acceptable carriers. The pharmaceutical composition may be therapeutic or preventive agents comprising said compounds and pharmaceutically acceptable carriers.

Diseases caused by the overproduction and the accumulation of ECM include glomerular nephritis, diabetic nephropathy or tissue fibrosis and they also include glomerular nephritis, diabetic nephropathy or tissue fibrosis that are derived from the abnormal proliferation of the mesangium cells.

As used herein, pharmaceutically acceptable carriers can include those that are identical with the excipients mentioned below. The amounts blended of a compound that inhibits the biological activity of galectin-3 and a carrier, without any limitation, follow the dosage of the active ingredient mentioned below, and can be widely selected. The amount of a compound that inhibits the biological activity of galectin-3 is usually 1 to 70 percent by weight and preferably 5 to 50 percent by weight in the total composition.

The composition thus obtained can be provided as an oral preparation such as a soft capsule, a hard capsule, a tablet, granules, powders, a suspension, a liquid, a syrup etc., an injection, a suppository, or an external preparation using a suitable excipient in a known method.

Such excipients include, for example, plant oils (for example, corn oil, cotton seed oil, coconut oil, almond oil, peanut oil, olive oil, and the like), oily esters such as glyceride oils of middle chain fatty acids, mineral oil, glycerin esters such as tricaprylin and triacetin, alcohols such as ethanol, physiological saline, propylene glycol, polyethylene glycol, vaseline, animal fats, cellulose derivatives (crystalline cellulose, hydroxypropyl cellulose, hydroxypropyl methyl cellulose, methyl cellulose), polyvinylpyrrolidone, cyclodextrin, dextrin, lactose, mannitol, sorbitol, starch and the like.

The dosage of the active ingredient, though depends on the degree of the disease and the age of the patient etc., is about 0.01 mg to 1000 mg per day per capita, preferably 1 mg to 200 mg per day per capita. It is desired that the formulations satisfy such conditions.

### EXAMPLES

The present invention will now be explained with reference to the following examples, but the present invention is not limited to these examples in any way.

### Example 1. Variation in the expression of galectin-3 in the rat nephritis model induced by anti-Thy-1.1 antibody

Rabbit anti-Thy-1.1 antiserum was obtained by immunizing rabbits subcutaneously on the back with Thy-1.1 antigen purified from rat thymus cells. The rat nephritis model induced by anti-Thy-1.1 antibody was prepared by intravenously administering to the tails of Sprague-Dawley rats 0.25 ml of the above rabbit anti-Thy-1.1 antiserum diluted 2-fold in phosphate buffered saline together with 0.25 ml of normal rabbit complement (Sigma) according to the method of Okuda et al. (Okuda S., et al., J. Clin. Invest. (1990) 86: 453-462). The rats were sacrificed on day 3, 7, and 14 after the administration of the antibody, and the kidney was extracted from each rat after perfusion with phosphate buffered saline. The extracted kidney was fixed in 4 % (w/v) phosphate buffered formalin, embedded in paraffin, and tissue sections for immunostaining were prepared. The immunostaining of the tissue sections was carried out by using affinity-purified rabbit anti-galectin-3 antibody as the primary antibody, peroxidase-labeled goat anti-rabbit antibody (Sigma) as the secondary antibody, and DAB (Sigma: DAB Tablet) as the chromogenic substrate.

The result of immunostaining of the tissue sections is shown in Figure 1. It was confirmed that in the rats who received no anti-Thy-1.1 antibody, a small quantity of galectin-3 was present in the distal urinary tubule and the macula densa region of the glomerulus, while in the rats who received anti-Thy-1.1 antibody, a large quantity of galectin-3 was observed in the distal and the proximal urinary tubule and the glomerulus on day 8 and 14. after the antibody administration. It was also confirmed that on day 14, infiltrated macrophage and fibroblasts were coexistent with galectin-3 in the pre-fibrotic region of the tubulo-interstitium. This finding confirmed that the expression of galectin-3 is increased at the time of pathogenesis in the rat nephritis models induced by anti-Thy-1.1 antibody, and since galectin-3 was coexistent with infiltrated macrophages or fibroblasts which are a kind of ECM-producing cells and are believed to induce the overproduction of ECM in the pre-fibrotic region, it was strongly suggested that galectin-3 is involved in the formation of fibrosis.

### Example 2. Variation in aalectin-3 expression in the unilateral ureteral obstruction (UUO)-treated rat interstitial fibrosis model

Female Sprague-Dawley rats (around 8 weeks old at the start of the experiment) were used. Under anesthesia with pentobarbital, complete ureteral obstruction of the left kidney was produced by ligating the ureter with 4-0 silk suture at two points and cutting between the ligatures. The left kidneys from each rat with UUO were harvested as obstructed kidneys and the right kidney as contralateral kidneys after perfused with phosphate buffered saline under diethyl ether anesthesia after 10 days from the operation. Harvested kidneys were fixed with 4 % (w/v) phosphate buffered paraformaldehyde for overnight and transferred to 70% ethanol. Fixed kidneys were embedded with paraffin and sectioned for immunostaining. The immunostaining of the tissue sections was carried out by using an affinity-purified rabbit anti-galectin-3 antibody as the primary antibody, a peroxidase-labeled goat anti-rabbit antibody (Sigma) as the secondary antibody, and DAB (Sigma: DAB Tablet) as the chromogenic substrate.

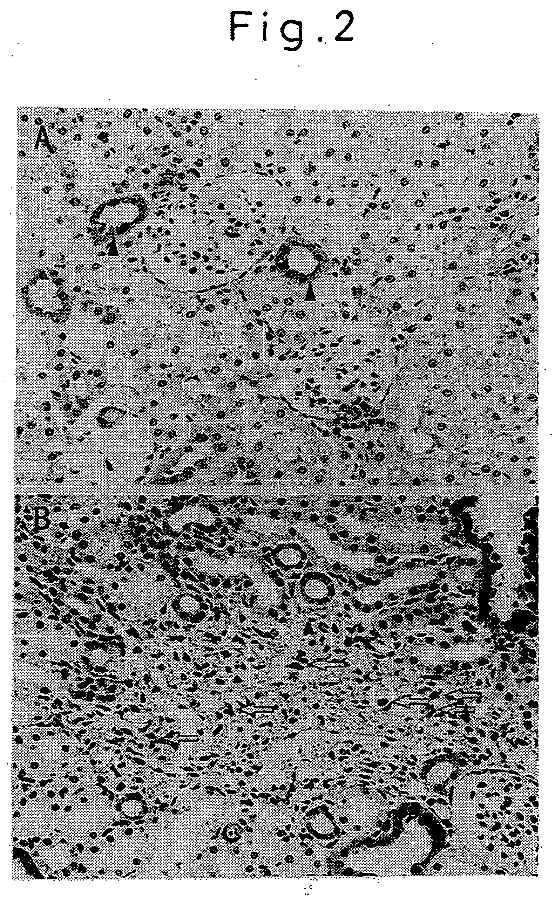

The result of immunostaining of the tissue sections is shown in Figure 2. The UUO model in which the overproduction and the accumulation of ECM such as collagen is observed in the interstitial region of the urinary tubule is a well known model that induces interstitial fibrosis (see Wright, E. J., et al., Lab. Invest. (1996) 74: 528-537, Yamate, J., et al., Toxicol. Pathol. (1998) 26: 793-801 and the like). In the contralateral kidneys, a small quantity of galectin-3 is present in the distal urinary tubule and the macula densa region of the glomerulus, while in the obstructed kidneys, infiltrated macrophage and fibroblasts were coexistent with galectin-3 in the fibrotic region of the tubulo-interstitium. This finding strongly suggested that galectin-3 is involved in the formation of fibrosis because galectin-3 was coexistent with infiltrated macrophage or fibroblasts which are a kind of ECM-producing cells and are believed to induce the overproduction of ECM in the fibrotic region.

### Example 3. Suppressive activity of galectin-3 on the cellular death of rat mesangium cells

Rat mesangium cells were separated from Sprague-Dawley rats according to the method of Striker et al. (Striker, G. E., et al., Lab. Invest. (1985) 53; 123-128). The separated rat mesangium cells were cultured at 37°C in the presence of 5% CO₂ in the wells of a 96-well plate using an essential medium (DMEM/F12 (1:1) culture medium containing 60 µg/ml penicillin, 100 µg/ml streptomycin, manufactured by Gibco BRL) supplemented with 10% fetal bovine serum. After culturing to semi-confluence, the cultured rat mesangium cells were washed with the essential medium, cultured for 2 days in the essential medium supplemented with 0.1% bovine serum albumin (Sigma), and then were further cultured for 1 to 4 days in the essential medium supplemented with 0.1% bovine serum albumin (Sigma) containing 0 or 50 µg/ml galectin-3 and 0 or 0.4 ng/ml TGF-β. On day 1, 2, 3, and 4 after the addition of galectin-3 and TGF-β, the amount of the cultured rat mesangium cells that survived in their respective wells were measured using as an index the conversion from MTS tetrazolium (Cell Titer 96 Aqueous one solution manufactured by Promega) to formazan by the living cells.

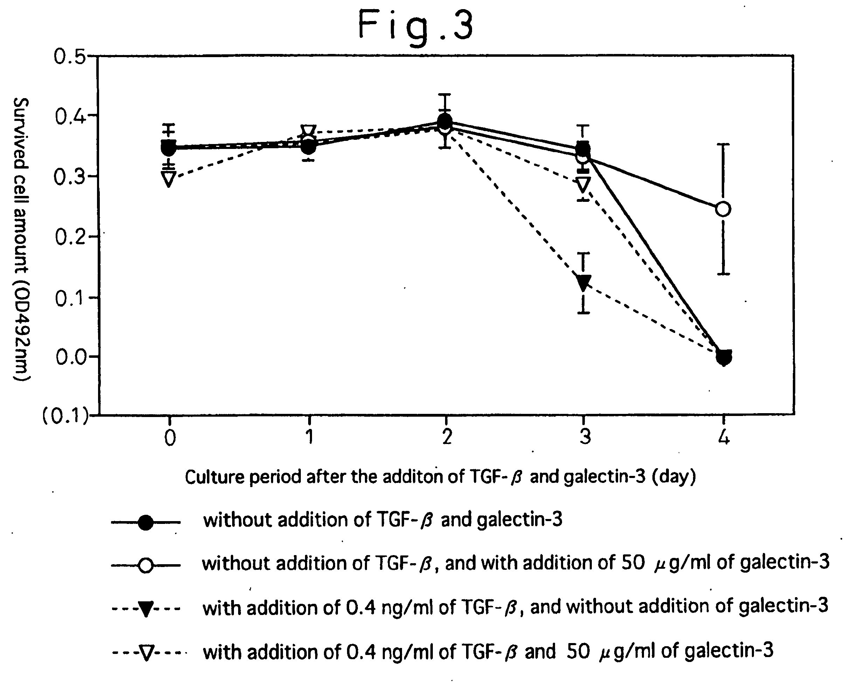

The result is shown in Figure 3. In both of the presence and the absence of TGF-β, galectin-3 was confirmed to suppress the cellular death of the rat mesangium cells, a kind of ECM-producing cells.

### Example 4. Promoting effect of galectin-3 on the collagen type IV production by mesangium cells

Rat mesangium cells were separated in a similar manner to that of Example 3. The separated mesangium cells were cultured at 37°C in the presence of 5% CO₂ in the wells of a 96-well plate using an essential medium (DMEM/F12 (1:1) culture medium containing 60 µg/ml penicillin, 100 µg/ml streptomycin, manufactured by Gibco BRL) supplemented with 10% fetal bovine serum. After culturing to confluence, the cultured rat mesangium cells were washed with the essential medium, cultured for 1 to 2 days in the essential medium supplemented with 0.1% bovine serum albumin (Sigma), and then were further cultured for 3 days in the essential medium supplemented with 0.1% bovine serum albumin (Sigma) containing 0, 10 or 30 µg/ml galectin-3 and 0, 0.1, 0.4 or 1.6 ng/ml TGF-β. On day 3 after the addition of galectin-3 and TGF-β, the amount of type IV collagen accumulated in the culture liquid was measured using a sandwich ELISA method that employed a goat anti-type IV collagen antibody as the immobilized antibody and a biotin-labeled goat anti-type IV collagen antibody (Chemicon) as the primary antibody. The amount of type IV collagen in the culture liquid of each well was normalized by dividing it by the amount of the living cells in each well determined in a similar manner to that described in Example 3.

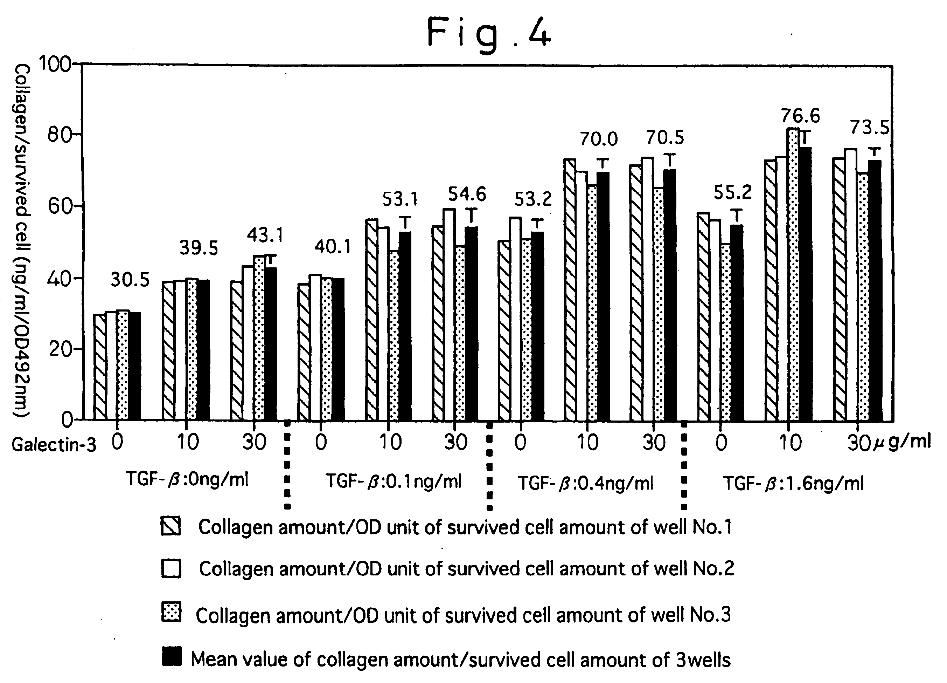

The result is shown in Figure 4. It was confirmed that galectin-3 promotes the production and/or the accumulation of type IV collagen which is a kind of ECM, from the rat mesangium cells which is a kind of ECM-producing cells, in a similar manner to and in an additive manner with TGF-β.

### Example 5. Inhibition of promotion by galectin-3 on collagen type IV production by rat mesangium cells, using a glycoprotein that inhibits galectin-3-binding

Rat mesangium cells were separated in a similar manner to that of Example 2. The separated rat mesangium cells were cultured at 37°C in the presence of 5% CO₂ in wells of a 96-well plate using an essential medium (DMEM/F12 (1:1) culture medium containing 60 µg/ml penicillin, 100 µg/ml streptomycin, manufactured by Gibco BRL) supplemented with 10% fetal bovine serum. After culturing to confluence, the cultured rat mesangium cells were washed with the essential medium, cultured for 1 to 2 days in the essential medium supplemented with 0.1% bovine serum albumin (Sigma), and then were further cultured for 4 days in the essential medium supplemented with 0.1% bovine serum albumin (Sigma) containing 10 µg/ml of galectin-3 and 0, 0.1, 0.2, 0.5 or 1.5 mg/ml fetuin glycoprotein which is a substance known to inhibit galectin-3 binding (see, for example, Sato, S. et al., J. Biol. Chem. (1992) 267: 6983-6990). On day 4 after the addition of galectin-3 and fetuin, the amount of type IV collagen accumulated in the culture liquid of each well was measured using a sandwich ELISA method that employed a goat anti-type IV collagen antibody (Chemicon) as the immobilized antibody and a biotin-labeled goat anti-type IV collagen antibody (Chemicon) as the primary antibody. The amount of type IV collagen in the culture liquid of each well was normalized by dividing it by the amount of the living cells in each well determined in a similar manner to that described in Example 3.

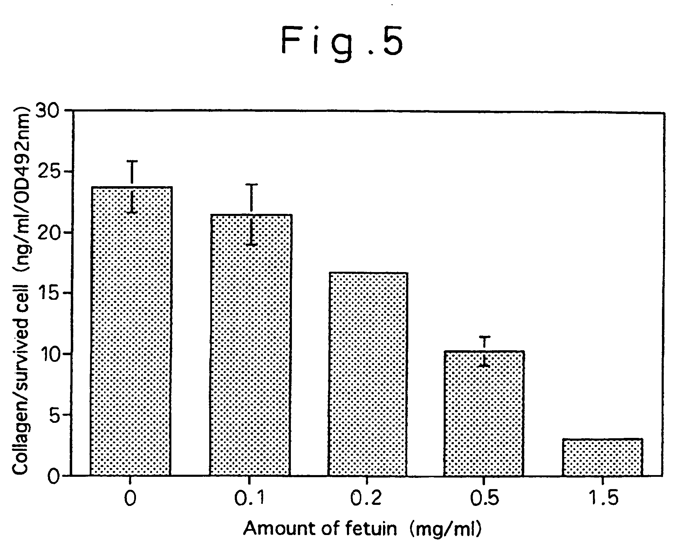

The result is shown in Figure 5. It was confirmed that a high molecular weight glycoprotein that inhibits galectin-3 binding suppresses the promotion of the production and/or the accumulation of type IV collagen which is a kind of ECM, from the rat mesangium cells which are a kind of ECM-producing cells, by the addition of galectin-3.

### Example 6. Inhibition, by galectin-3-binding inhibiting sugar, of the effect of galectin-3 on the promotion of collagen type IV production by rat mesangium cells

Rat mesangium cells were separated in a similar manner to that described in Example 3. The separated rat mesangium cells were cultured at 37°C in the presence of 5% CO₂ in the wells of a 96-well plate using an essential medium (DMEM/F12 (1:1) culture medium containing 60 µg/ml penicillin, 100 µg/ml streptomycin, manufactured by Gibco BRL) supplemented with 10% fetal bovine serum. After culturing to confluence, the cultured rat mesangium cells were washed in the essential medium, cultured for 1 to 2 days with the essential medium supplemented with 0.1% bovine serum albumin (Sigma), and then were further cultured for 2 days in the essential medium supplemented with 0.1% bovine serum albumin (Sigma) containing 0.4 µg/ml of galectin-3 and 0, 0.25, 0.5, 1 or 2 mM of lacto-n-fucopentaose I which is, a substance known to inhibit galectin-3 binding (LNFP-1, see, for example, Sato, S. et al., J. Biol. Chem. (1992) 267: 6983-6990). On day 2 after the addition of galectin-3 and LNFP-I, the amount of type IV collagen accumulated in the culture liquid of each well was measured using a sandwich ELISA method that employed a goat anti-type IV collagen antibody (Chemicon) as an immobilized antibody and a biotin-labeled goat anti-type IV collagen antibody (Chemicon) as a primary antibody. The amount of type IV collagen in the culture medium of each well was normalized by dividing it by the amount of the living cells in each well determined in a similar manner to that described in Example 4.

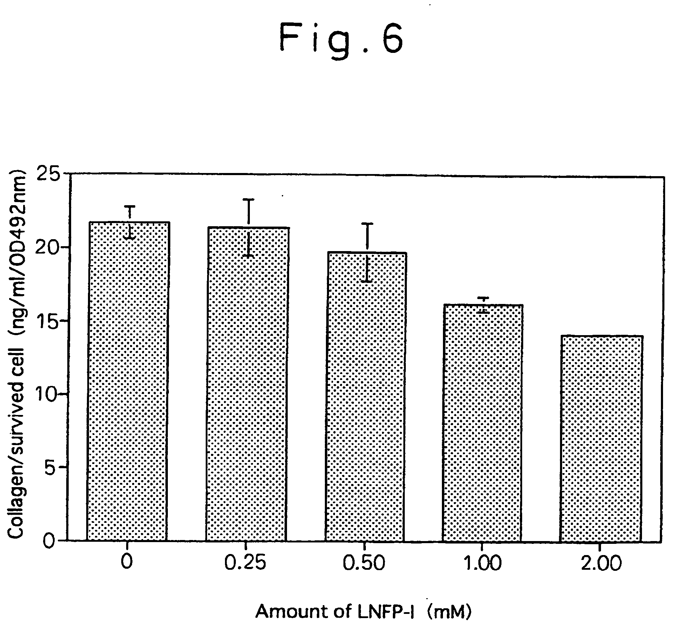

The result is shown in Figure 6. It was confirmed that a low molecular weight sugar that inhibits galectin-3 binding suppresses the promotion of the production and/or the accumulation of type IV collagen which is a kind of ECM, from the rat mesangium cells which are a kind of ECM-producing cells, by the addition of galectin-3.

As hereinabove described, it was shown that galectin-3 exhibits an increased expression during pathogenesis in the anti-Thy-1.1 antibody-induced rat nephritis model, an animal model of mesangial proliferative glomerulonephritis, (Example 1), and thus it was suggested that galectin-3 is involved in the pathogenesis of mesangial proliferative glomerulonephritis. It was also demonstrated that in the anti-Thy-1.1 antibody-induced rat nephritis model and in the obstructed kidneys of the UUO rat model, galectin-3 is coexistent with infiltrated macrophage and fibroblasts in the pre-fibrotic or fibrotic region of the tublo-interstitium (Examples 1 and 2). This finding strongly suggested that galectin-3 is involved in the formation of fibrosis because galectin-3 was coexistent with infiltrated macrophage and/or fibroblasts, a kind of ECM-producing cells, that are believed to induce the overproduction of ECM in the pre-fibrotic or fibrotic region. It was also demonstrated that galectin-3 inhibits the cellular death of the mesangium cells, a kind of ECM-producing cells (Example 3), and that it promotes the production and/or the accumulation of ECM from the ECM-producing cells (Example 4). It was further shown that a substance that inhibits the biological activity of galectin-3 suppresses the promotion of the production and/or the accumulation of ECM from ECM-producing cells by galectin-3 (Examples 5 and 6).

### INDUSTRIAL APPLICABILITY

The use of the present invention can be clinically applicable as a therapeutic or preventive agent for glomerular nephritis, diabetic nephropathy or tissue fibrosis.

## Claims

1. The use of a compound that binds galectin-3 in the manufacture of a medicament for the treatment of glomerular nephritis, diabetic nephropathy or tissue fibrosis, wherein the compound that binds galectin-3 is an inhibitor of galectin-3 binding and is selected from:
i) a sugar to which galectin-3 can bind;
ii) a blood type B-like sugar chain;
iii) a glycolipid comprising a sugar to which galectin-3 can bind;
iv) a glycoprotein comprising a sugar to which galectin-3 can bind;
v) a fragment of the glycoprotein of part iv); and
vi) an anti-galectin-3 antibody.

2. The use of claim 1, wherein the sugar of part i), iii) or iv) is selected from the group consisting of: Galβ1-4Glc, Galβ1-4GlcNAc, Fucα1-2Galβ1-4Glc, Galα1-3Galβ1-4GlcNAc, Galβ1-3GlcNAcβ-1-3GaLβ1-4Glc, Galβ1-4GlcNAcβ1-3Galβ1-4Glc, Fucα1-2Galβ1-3GlcNAcβ1-3Galβ1-4Glc, Galβ-3(Fucα1-4)GlcNAcβ1-3Galβ1-4Glc, Galβ1-4 (Fucα1-3)GlcNAcβ1-3Galβ1-4Glc, Galβ1-3GlcNAcβ1-3Galβ1-4 (Fucα1-3)Glc, Fucα1-2(GlcNAcα1-3)Galβ1-3GlcNAcβ1-3Galβ1-4Glc, NeuNAcα2-3Galβ1-3GlcNAcβ1-3Galβ1-4Glc, NeuNAcα2-6-Galβ1-4GlcNAcβ1-3Galβ1-4Glc, Galβ-3(NeuNAcα2-6)GlcNAcβ1-3Ga1β1-4Glc, Galβ1-3-GleNAcβ1-3Galβ1-4GlcNAcβ-3Galβ1-4Glc, Galβ1-4GlcNAcβ1-3Galβ1-4GlcNAcβ1-3Galβ1-4Glc, Galβ1-3GlcNAcβ1-3Galβ1-4(Fucα1-3)GlcNAcβ1-3Galβ1-4Glc, Galβ1-4GlcNAcβ1-6(Galβ1-3GlcNAcβ1-3)Galβ1-4Glc, Galβ1-4GlcNAcβ1-6(Galβ1-4GlcNAcβ-3)Galβ1-4Glc, Galβ1-4GlcNAcβ1-6(Galβ1-4GlcNAcβ1-2)Manα1-6(Galβ1-4GlcNAcβ1-2Manα1-3)Manβ1-4GlcNAc, Galβ1-4GlcNAcβ1-2Manα1-6(Galβ1-4GlcNAcβ1-4(Galβ1-4GlcNAcβ1-2)Manα1-3)Manβ1-4GlcNAc, GlcNAcβ1-3Galβ1-4GlcNAcβ1-3Galβ1-4Glc, Galα1-3Galβ1-4GlcNAcβ1-3Galβ1-4Glc, GalNAcα1-3(Fucα1-2)Galβ1-3GlcNAcβ1-3Galβ1-4Glc, Galα1-3(Fucα1-2)Galβ1-3GlcNAcβ1-3Galβ1-4Glc, Galβ1-4GlcNAcβ1-6(Galβ1-4GlcNAcβ1-3)Galβ1-4GlcNAcβ1-3Galβ1-4Glc, Galα1-3Galβ1-4GlcNAcβ1-6(Galα1-3Galβ1-4GlcNAcβ1-3)Galβ1-4GlcNAcβ1-3Galβ1-4Glc, Galβ1-4GlcNAcβ1-6(Galβ1-4GlcNAcβ1-3)Galβ1-4GlcNAcβ1-6(Galβ1-4GlcNAcβ1-3)Galβ1-4GlcNAcβ1-3Galβ1-4Glc, Galα1-3Galβ1-4GlcNAcβ1-6(Galα1-3Galβ1-4GlcNAcβ1-3)Galβ1-4GlcNAcβ1-6(Galα1-3Galβ1-4GlcNAcβ1-3)Galβ1-4GlcNAcβ1-3Galβ1-4Glc, Galβ1-4GlcNAcβ1-2Manα1-6(Galβ1-4GlcNAcβ1-2Manα1-3)Manβ1-4GlcNAc, Galβ1-4GlcNAcβ1-2Manα1-6(Galβ1-4GlcNAcβ1-4(Galβ1-4GlcNAcβ1-2)Manα1-3)Manβ1-4GlcNAc, and Galβ1-4GlcNAcβ1-6(Galβ1-4GlcNAcβ1-2)Manα1-6(Galβ1-4GlcNAcβ1-4(Galβ1-4GlcNAcβ1-2)Manα1-3)Manβ1-4GlcNAc.

3. The use of claim 1, wherein the glycoprotein of part iv) is selected from the group consisting of: fetuin, asialofetuin, transferrin, asialotransferrin, α1-acid glycoprotein, asialo α1-acid glycoprotein, laminin, fibronectin, CDIIb, Lamp-1, Lamp-2, Mac-3, CD98, neutrophil 115 kD protein, neutrophil 180 kD protein (NCA-160/CD66), high affinity IgE receptor, or Fc ε R1.

4. The use of any of claims 1 to 3, wherein the glomerular nephritis, diabetic nephropathy or tissue fibrosis is caused by the abnormal proliferation of mesangium cells.

5. The use of any of claims 1 to 4, wherein said treatment is therapeutic.

6. The use of any of claims 1 to 4, wherein said treatment is preventive.

## Patentansprüche

1. Verwendung einer Verbindung, die Galektin-3 bindet, bei der Herstellung eines Medikaments zur Behandlung von glomerularer Nephritis, diabetischer Nephropathie oder Gewebefibrose, wobei die Verbindung, die Galektin-3 bindet, ein Inhibitor der Galektin-3-Bindung ist und ausgewählt ist aus:
i) einem Zucker, an den Galektin-3 binden kann;
ii) einer Blutgruppe-B-artigen Zuckerkette;
iii) einem Glykolipid, umfassend einen Zucker, an den Galektin-3 binden kann;
iv) einem Glykoprotein, umfassend einen Zucker, an den Galektin-3 binden kann;
v) einem Fragment des Glykoproteins von Bestandteil iv); und
vi) einem Anti-Galektin-3-Antikörper.

2. Verwendung nach Anspruch 1, wobei der Zucker von Bestandteil i), iii) oder iv) ausgewählt ist aus der Gruppe bestehend aus: Galβ1-4Glc, Galβ1-4GlcNAc, Fucα1-2Galβ1-4Glc, Galα1-3Galβ1-4GlcNAc, Galβ1-3GlcNAcβ-1-3Galβ1-4Glc, Galβ1-4GlcNAcβ1-3Galβ1-4Glc, Fucα1-2Galβ1-3GlcNAcβ1-3Galβ1-4Glc, Galβ-3(Fucα1-4)GlcNAcβ1-3Galβ1-4Glc, Galβ1-4(Fucα1-3)GlcNAcβ1-3Galβ1-4Glc, Galβ1-3GlcNAcβ1-3Galβ1-4(Fucα1-3)Glc, Fucα1-2(GlcNAcα1-3)Galβ1-3GlcNAcβ1-3Galβ1-4Glc, NeuNAcα2-3Galβ1-3GlcNAcβ1-3Galβ1-4Glc, NeuNAcα2-6-Galβ1-4GlcNacβ1-3Galβ1-4Glc, Galβ-3(NeuNAcα2-6)GlcNAcβ1-3Galβ1-4Glc, Galβ1-3-GlcNAcβ1-3Galβ1-4GlcNAcβ1-3Galβ1-4Glc, Galβ1-4GlcNAcβ1-3Galβ1-4GlcNAcβ1-3Galβ1-4Glc, Galβ1-3GlcNAcβ1-3Galβ1-4(Fucα1-3)GlcNAcβ1-3Galβ1-4Glc, Galβ1-4GlcNAcβ1-6(Galβ1-3GlcNAcβ1-3)Galβ1-4Glc, Galβ1-4GlcNAcβ1-6(Galβ1-4GlcNAcβ1-3)Galβ1-4Glc, Galβ1-4GlcNAcβ1-6(Galβ1-4GlcNAcβ1-2)Manα1-6(Galβ1-4GlcNAcβ1-2Manα1-3)Manβ1-4GlcNAc, Galβ1-4GlcNAcβ1-2Manα1-6(Galβ1-4GlcNAcβ1-4(Galβ1-4GlcNAcβ1-2)Manα1-3)Manβ1-4GlcNAc, GlcNAcβ1-3Galβ1-4GlcNAcβ1-3Galβ1-4Glc, Galα1-3Galβ1-4GlcNAcβ1-3Galβ1-4Glc, GalNAcα1-3(Fucα1-2)Galβ1-3GlcNAcβ1-3Galβ1-4Glc, Galα1-3(Fucα1-2)Galβ1-3GlcNAcβ1-3Galβ1-4Glc, Galβ1-4GlcNAcβ1-6(Galβ1-4GlcNAcβ1-3)Galβ1-4GlcNAcβ1-3Galβ1-4Glc, Galα1-3Galβ1-4GlcNAcβ1-6(Galα-3Galβ1-4GlcNAcβ1-3)Galβ1-4GlcNAcβ1-3Galβ1-4Glc, Galβ1-4GlcNAcβ1-6(Galβ1-4GlcNAcβ1-3)Galβ1-4GlcNAcβ1-6(Galβ1-4GlcNAcβ1-3)Galβ1-4GlcNAcβ1-3Galβ1-4Glc, Galα1-3Galβ1-4GlcNAcβ1-6(Galα1-3Galβ1-4GlcNAcβ1-3)Galβ1-4GlcNAcβ1-6(Galα1-3Galβ1-4GlcNAcβ1-3)Galβ1-4GIcNAcβ1-3Galβ1-4Glc, Galβ1-4GlcNAcβ1-2Manα1-6(Galβ1-4GlcNAcβ1-2Manα1-3)Manβ1-4GlcNAc, Galβ1-4GlcNAcβ1-2Manα1-6(Galβ1-4GlcNAcβ1-4(Galβ1-4GlcNAcβ1-2)Manα1-3)Manβ1-4GlcNAc, und Galβ1-4GlcNAcβ1-6(Galβ1-4GlcNAcβ1-2)Manal-6(Galβ1-4GlcNAcβ1-4(Galβ1-4GlcNAcβ1-2)Manα1-3)Manβ1-4GlcNAc.

3. Verwendung nach Anspruch 1, wobei das Glykoprotein von Bestandteil iv) ausgewählt ist aus der Gruppe bestehend aus: Fetuin, Asialofetuin, Transferrin, Asialotransferrin, α1-Säure-Glykoprotein, Asialo-α1-Säure-Glykoprotein, Laminin, Fibronektin, CD11b, Lamp-1, Lamp-2, Mac-3, CD98, neutrophilem 115-kD-Protein, neutrophilem 180-kD-Protein (NCA-160/CD66), hochaffinem IgE-Rezeptor oder Fc ε R1.

4. Verwendung nach einem der Ansprüche 1 bis 3, wobei die glomerulare Nephritis, diabetische Nephropathie oder Gewebefibrose durch die abnorme Proliferation von Mesangiumzellen verursacht ist.

5. Verwendung nach einem der Ansprüche 1 bis 4, wobei die Behandlung therapeutisch ist.

6. Verwendung nach einem der Ansprüche 1 bis 4, wobei die Behandlung präventiv ist.

## Revendications

1. Utilisation d'un composé liant la galectine-3 dans la fabrication d'un médicament pour le traitement de la néphrite glomérulaire, de la néphropathie diabétique et de la fibrose des tissus, dans laquelle le composé liant la galectine-3 est un inhibiteur de liaison à la galectine-3 et est choisi parmi :
i) un sucre avec lequel la galectine-3 peut se lier
ii) une chaîne de sucre du sang d'un type similaire au B ;
iii) un glycolipide comprenant un sucre avec lequel la galectine-3 peut se lier ;
iv) une glycoprotéine comprenant un sucre avec lequel la galectine-3 peut se lier ;
v) un fragment de la glycoprotéine selon la partie iv) ; et
vi) un anticorps anti-galectine-3

2. utilisation selon la revendication 1, dans laquelle le sucre selon la partie i), iii), ou iv) est choisi dans le groupe consistant en : Galβ1-4Glc, Galβ1-4GlcNAc, Fucα1-2Galβ1-4Glc, Galα1-3Galβ1-4GlcNAc, Galβ1-3GlcNAcβ-1-3Galβ1-4Glc, Galβ1-4GlcNAcβ1-3Galβ1-4Glc, Fucα1-2Galβ1-3GlcNAcβ1-3Galβ1-4Glc, Galβ-3(Fucα1-4)GlcNAcβ1-3Galβ1-4Glc, Galβ1-4(Fucα1-3)GlcNAcβ1-3Galβ1-4Glc, Galβ1-3GlcNAcβ1-3Galβ1-4(Fucα1-3)Glc, Fucα1-2(GlcNAcα1-3)Galβ1-3GlcNAcβ1-3Galβ1-4Glc, NeuNAcα2-3Galβ1-3GlcNAcβ1-3Galβ1-4Glc, NeuNAcα2-6-Galβ1-4GlcNAcβ1-3Galβ1-4Glc, Galβ-3(NeuNAcα2-6)GlcNAcβ1-3Galβ1-4Glc, Galβ1-3-GlcNAcβ1-3Galβ1-4GlcNAcβ1-3Galβ1-4Glc, Galβ1-4GlcNAcβ1-3Galβ1-4GlcNAcβ1-3Galβ1-4Glc, Galβ1-3GlcNAcβ1-3Galβ1-4(Fucα1-3)GlcNAcβ1-3Galβ1-4Glc, Galβ1-4GlcNAcβ1-6(Galβ1-3GlcNAcβ1-3)Galβ1-4Glc, Galβ1-4GlcNAcβ1-6(Galβ1-4GlcNAcβ1-3)Galβ1-4Glc, Galβ1-4GlcNAcβ1-6(Galβ1-4GlcNAcβ1-2)Manα1-6(Galβ1-4GlcNAcβ1-2Manα1-3)Manβ1-4GlcNAc, Galβ1-4GlcNAcβ1-2Manal-6(Galβ1-4GlcNAcβ1-4(Galβ1-4GlcNAcβ1-2)Manα1-3)Manβ1-4GlcNAc, GlcNAcβ1-3Galβ1-4GlcNAcβ1-3Ga1β1-4Glc, Galα1-3Galβ1-4GlcNAcβ1-3Galβ1-4Glc, GalNAcα1-3(Fucα1-2)Galβ1-3GlcNAcβ1-3Galβ1-4Glc, Galα1-3(Fucα1-2)Galβ1-3GlcNAcβ1-3Ga1β1-4Glc, Galβ1-4GlcNAcβ1-6(Galβ1-4GlcNAcβ1-3)Galβ1-4GlcNAcβ1-3Galβ1-4Glc, Galα1-3Galβ1-4GlcNAcβ1-6(Galα1-3Ga1β1-4GlcNAcβ1-3)Galβ1-4GlcNAcβ1-3Galα1-4Glc, Galβ1-4GlcNAcβ1-6(Galβ1-4GlcNAcβ1-3)Galβ1-4GlcNAcβ1-6(Galβ1-4GlcNAcβ1-3)Galβ1-4GlcNAcβ1-3Galβ1-4Glc, Galα1-3Galβ1-4GlcNAcβ1-6(Galα1-3Galβ1-4GlcNAcβ1-3)Galβ1-4GlcNAcβ1-6(Galα1-3Galβ1-4GlcNAcβ1-3)Galβ1-4GlcNAcβ1-3Galβ1-4Glc, Galβ1-4GlcNAcβ1-2Manα1-6(Galβ1-4GlcNAcβ1-2Manα1-3)Manβ1-4GlcNAc, Galβ1-4GlcNAcβ1-2Manα1-6(Galβ1-4GlcNAcβ1-4(Galβ1-4GlcNAcβ1-2)Manα1-3)Manβ1-4GlcNAc, et Galβ1-4GlcNAcβ1-6(Galβ1-4GlcNAcβ1-2)Manα1-6(Galβ1-4GlcNAcβ1-4(Galβ1-4GlcNAcβ1-2)Manα1-3)Manβ1-4GlcNAc.

3. utilisation selon la revendication 1, dans laquelle la glycoprotéine selon la partie iv) est choisie dans le groupe consistant en : la fetuine, l'asialofetuine, la transferrine, l'asialotransferrine, l'α-1 glycoprotéine acide, l'α-1 asialoglycoprotéine acide, la laminine, la fibronectine, le CD11b, le Lamp-1, le Lamp-2, le Mac-3, le CD98, la protéine de neutrophile 115kD, la protéine de neutrophile 180kD (NCA-160/CD66), un récepteur de haute affinité pour les IgE, ou FcεRI.

4. Utilisation selon l'un quelconque des revendications 1 à 3, dans laquelle la néphrite glomérulaire, la neuropathie diabétique ou la fibrose de tissus sont causées par la prolofération anormale de cellules mesangiales.

5. Utilisation selon l'une quelconque des revendications 1 à 4, dans laquelle ledit traitement est thérapeutique.

6. Utilisation selon l'une quelconque des revendications 1 à 4, dans laquelle ledit traitement est préventif.
